# EUROPEAN PATENT SPECIFICATION

(11) **EP 1 163 002 B1**
(45) Date of publication and mention of the grant of the patent: **06.08.2008**
(21) Application number: 00912788.7
(22) Date of filing: 23.03.2000
(51) Int. Cl.: A61K 39/39, A61K 39/02, A61K 39/05, A61K 9/127, A61K 9/50, A61P 31/04

(54) **POLYCATIONIC CARBOHYDRATES AS IMMUNOSTIMULANTS IN VACCINES**
POLYKATIONISCHE KARBOHYDRATE ALS IMMUNOSTIMULIERENDE MITTEL IN IMPSTOFFEN
CARBOHYDRATES POLYCATIONIQUES COMME IMMUNOSTIMULANTS DANS DES VACCINS

(30) Priority: 24.03.1999 GB 9906696; 24.03.1999 GB 9906694
(43) Date of publication of application: 19.12.2001
(73) Proprietor: THE SECRETARY OF STATE FOR DEFENCE, Salisbury Wiltshire SP4 0JQ (GB)
(72) Inventor: ALPAR, Hazire Oya, Birmingham B4 7ET (GB); EYLES, James Edward, Birmingham B4 7ET (GB); SOMAVARAPU, Satyanarayana, Birmingham B4 7ET (GB); WILLIAMSON, Ethel Diane, Salisbury - Wiltshire SP4 0JQ (GB); BAILLIE, Leslie William James, Salisbury Wiltshire SP4 0JQ (GB)
(74) Representative: Beckham, Robert William
(86) International application number: PCT/GB2000/001118
(87) International publication number: WO 2000/056362

(56) References cited:
- WO-A-96/10421
- WO-A-97/20576
- US-A- 5 585 106
- KOTZE AWIE F ET AL: "Enhancement of paracellular drug transport with highly quaternized N-trimethyl chitosan chloride in neutral environments: In vitro evaluation in intestinal epithelial cells (Caco-2)." JOURNAL OF PHARMACEUTICAL SCIENCES, vol. 88, no. 2, February 1999 (1999-02), pages 253-257, XP000792263 ISSN: 0022-3549

## Description

The present invention relates to compounds which are polycationic carbohydrates, for use as immunostimulants and to vaccines containing these. The invention further comprises the use of the compounds in the manufacture of a vaccine for protecting an animal against infection by a pathogen as well as methods of preparing the compositions.

A prime objective in the field of vaccination is the development of a non-parenteral immunisation regimen, which facilitates induction of comparable levels of systemic immunity to that elicited by conventional sub-cutaneous and intra-muscular injections.

The nasopharyngeal passages and pulmonary regions of the respiratory tract represent potential targets for the systemic delivery of peptidergic drugs and vaccines. The relative ease with which therapeutic agents can be inhaled, or introduced into the nose, make these modes of immunisation attractive in terms of probable patient compliance. Furthermore, respiratory mucosae offer certain morphological, physiological and immunological advantages over other non-parenteral sites in terms of immunisation, particularly against pathogenic entities which affect or utilise mucosal surfaces as portals of entry. This is because effective vaccination against these pathogens normally requires mucosae to be adequately protected with locally produced antibodies of the secretory IgA (sIgA) isotype. Whilst mucosal surfaces are usually poorly protected with IgA following parenteral administration of vaccines, it is now apparent that successful delivery of antigenic material to immunoresponsive elements in mucosa-associated lymphoid tissue (MALT) can result in vigorous stimulation of the mucosal arm of the immune system. By means of the common mucosal immune system (CMIS) it is feasible that several anatomically disparate mucosal surfaces could be protected through mucosal administration of a vaccine at a single site. Mucosal vaccination offers the added advantage that some degree of systemic immunity can be induced in concert with local responses due to translocation of antigenic material from sub-epithelial compartments to systemic immunoresponsive tissues such as the spleen.

Despite the logistical and immunological factors which favour non-parenteral immunisation, simple mucosal application of antigenic proteins, for example in the gastrointestinal or respiratory tracts, is usually ineffectual in terms of vaccination. Enzymatic or chemical destruction, combined with poor absorption into sub-epithelial compartments dictate that mucosally administered vaccines usually require some form of adjuvant or delivery vehicle. One approach is to encapsulate antigenic material within microparticulate polymeric carriers, such as poly-DL-lactide (PLA) microspheres (Vaccine 1994, 12, 5-11). Such procedures serve to protect labile vaccines from lumenal degradation and enhance absorption into mucosal and systemic compartments (J.H. Eldridge et al., Seminars in Hematology, (1993), 30, 16-25). There is good evidence that microencapsulation may also adjuvantise by converting soluble antigenic molecules into particulate species, thus promoting vaccine uptake into antigen presenting cells (APC)(Y. Tabata et al., Adv. Polym. Sci. (1990), 94, 107-141, L. Vidard et al., J. Immunol. (1996), 156, 2809-2818, N. Van Rooijen, Immunol. Today (1990) 11, 436-439) or microfold cells (M-cells) in lymphoid follicles (R.I. Walker et al., Vaccine, 12, 387, 1994, D.T. O'Hagan et al,, Vaccine, 1989, 7, 421-424, P.G. Jenkins et al., J. Drug Targetting, 1995, 3, 79-81).

Although comparatively under-investigated, the intra-nasal (i.n.) route is an attractive one for the mucosal delivery of vaccinal entities. The nasal epithelium is accessible and is less exclusive to high molecular weight molecules.

The thickness of the mucus blanket covering respiratory epithelium is relatively thin compared to that of other mucosae, for example the gut where it is in the region of 500 times thicker. Substantially reduced concentrations of proteolytic enzymes and extremes of pH exist in the respiratory tract compared with the gastrointestinal tract.

Furthermore, it is now delineated that nasal associated lymphoids tissues (NALT) have a lymphoepithelium which, like that in the intestinal mucosa, contain M-cells for selective antigen uptake (P. Brandenburg, Immunology of the Lung and Upper Respiratory Tract, (ed. Bienenstock J.) McGraw-Hill, New York, 1984, 28-95). Hence NALT plays an analogous role to other MALT, such as the gut associated lymphoid tissues (GALT), in terms of antigen surveillance and induction of mucosal and systemic immunological responses.

Polycationic carbohydrates such as chitosan as well as various derivatives or composites of these polymers have been used previously in the pharmaceutical compositions as absorption enhancers to increase delivery of an active pharmaceutical agent across a barrier such as a mucosal membrane. The applicants have found however, that when these compounds are used in the context of vaccine compositions, they act as adjuvants, producing an increase in the immune response to the antigenic agent being delivered. The increase in the response levels persists for considerably longer than would be expected as a result only of absorption enhancing effects.

WO 97/20576 discloses a vaccine composition for intra nasal administration comprising an antigen and an effective adjuvant amount of a chitosan; chitosan glutamate.

WO 96/10421 discloses a vaccine composition for intra nasal administration comprising an influenza virus antigen and an effective adjuvant amount of a chitosan.

Thus according to a first aspect of the present invention there is provided a polycationic carbohydrate or a pharmaceutically acceptable derivative thereof for use as an immunostimulant, wherein the polycationic carbohydrate comprises a water-soluble alkylated chitosan derivative or salt thereof.

As used herein, the term "immunostimulant" refers to an adjuvant which stimulates the immune system of a host animal to which it is administered and thereby increases the protective effect produced by a protective antigen administered to that animal, as compared to the effect which would be produced by administration of the protective antigen alone.

The expression "polycationic carbohydrate" includes polymeric compounds with repeat units of general formula Cₓ(H₂O)_{y}. They will include multiple cationic functions or groups such as amino or amido groups which are cationic in nature in that they will readily form cations such as quaternary ammonium compounds.

Particular examples of polycationic carbohydrates include immunostimulant chitin derivatives such as chitosans or chemically modified forms such as soluble forms thereof; or mixtures thereof with cationic polypeptides, cationic polyamino acids or quaternary ammonium compounds.

Examples of water-soluble derivatives of polycationic carbohydrates in particular, are water-soluble chitin derivatives such as an alkylated chitosan derivatives and salts thereof.

Examples of these chemicals include trimethyl chitosan chloride, carboxymethyl chitosan, N-carboxymethyl chitosan, and polyethylene glycol chitosan. N-trimethyl chitosan chloride (TMC) has been referred (Kotze, A. F. et al. Pharm Res. (1997) 14:1197-1202) as a potential absorption enhancer of peptide therapies across mucosal membranes. In contrast to chitosan, TMC is water soluble in all gastrointestinal pH environments. Further, it retains the ability to temporarily open tight junctions. An important parameter appears to be the degree of trimethylation.

Examples of biologically active agents which are capable of generating a protective immune response in an animal, particularly a mammal, and which may benefit from combined use with the immunostimulants of the invention are well known in the art. They include antigenic polypeptides as well as nucleic acid sequences which may encode these polypeptides and which are known as "naked DNA" vaccines. Live vaccines such as DNA or RNA viral vaccines may also be used in conjunction with the immunostimulant of the invention.

As used herein the expression "polypeptide" encompasses proteins or epitopic fragments thereof.

Suitable polypeptides are sub-unit vaccines, such as tetanus toxoid, diptheria toxoid and *Bacillus anthracis* protective antigen (PA).

A particular example of a biologically active agent is one which is capable of generating a protective immune response against *Yersinia pestis.* The agent is suitably a sub-unit vaccine, for example as described in WO 96/28551. The vaccine described and claimed there comprises a combination of the V antigen of *Y*. *pestis* or an immunologically active fragment thereof or a variant of these, and the F1 antigen of *Y. pestis* or an immunologically active fragment thereof or a variant of these.

As used herein, the term "fragment" refers to a portion of the basic sequence which includes at least one antigenic determinant. These may be deletion mutants. One or more epitopic region of the sequence may be joined together.

The expression "variant" refers to sequences of nucleic acids which differ from the base sequence from which they are derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type. Broadly speaking, fewer non-conservative substitutions will be possible without altering the biological activity of the polypeptide. Suitably variants will be at least 60% homologous, preferably at least 75% homologous, and more preferably at least 90% homologous to the base sequence. Homology in this instance can be judged for example using the algorithm of Lipman-Pearson, with Ktuple:2, gap penalty:4, Gap Length Penalty:12, standard PAM scoring matrix (Lipman, D.J. and Pearson, W.R., Rapid and Sensitive Protein Similarity Searches, Science, 1985, vol. 227, 1435-1441).

Previously, we have demonstrated that the intranasal (IN) route is a highly effective, non-invasive alternative, to the parenteral administration of recombinant subunit vaccines (F1 and V derived from the causative organism of plague: *Yersinia pestis*) (Eyles, J. E. et al. Vaccine (1998) 16:698-707; J. Drug Targeting Nasal delivery of vaccines) 1996 Alpar & Almeida). Fraction 1 (F1) subunit (molecular mass 15.5 kDa) is derived from the capsule that surrounds the bacteria. In solution, because of its hydrophobic nature, F1 tends to aggregate into multimeric complexes of high (>200,000 kDa) molecular weight (Voronisov, E. D. et al. Biomed. Sci. (1990) 1:391-396 and Miller, J. et al. FEMS Immun. Med. Micro. (1998) 21:213-231). The V antigen (molecular mass 37 kDa) is a protein secreted by the bacterium at 37°C (Leary, S. E. C. et al. Infect. Immun. (1995) 63:2854-2858). V is a virulence factor which may exert local anti-inflammatory effects through modulation of tissue cytokine levels (Nakajima, R. and R. R. Brubaker Infect. Immun. (1993) 61:23-31). Both F1 and V are protective, and there is a documented 'synergistic effect' in combination (Williamson, E. D. et al. Vaccine (1996) 14:1613-1619). Traditionally killed whole cell vaccines for plague have an unsatisfactory incidence of transient local and systemic side effects, but more importantly, may fail to protect individuals from the pneumonic form of the disesase, which is transmissible via airborne droplets (Perry, R. D. and J. D. Fetherston. Clin. Microbiol. Rev. (1997) 10:35-66). Intramuscular injection (Williamson, E. D. et al. Vaccine (1997) 15:1079-1084) of combined F1 and V, or intranasal administration of microspheres co-encapsulating F1 and V (Eyles, J. E. et al. Vaccine (1998) 16:698-707), can protect experimental animals against a lethal inhalational challenge with *Y. pestis.*

Following these experiments it was seen that systemic immunity, in the form of high serum IgG titres to F1 and V, was critical for protection. In the current work, the applicants have nasally instilled F1 (5 µg) and V (1 µg) in the presence and absence of a variety of chemicals including three different TMC derivatives (with increasing degrees of quaternization: 20, 40 and 60%), and compared humoral immune response engendered by these treatments to those evoked by co-administration of chitosan itself. The results indicate that the higher the degree of derivitisation, the greater the increase in the immune response. Thus TMC 60 is the most preferred form.

In a further aspect, the invention provides the use of a polycationic carbohydrate of the present invention as an immunostimulant in the preparation of a vaccine for use in protecting an animal against a pathogen, by administering to an animal a protective agent which is able to stimulate the animal's immune system to produce a response which is protective against the pathogen.

Suitable animals are mammals including humans.

The adjuvant or immunostimulant may be administered simultaneously with said protective agent, suitably in a combined formulation. Alternatively, it may be administered separately. The selection of particular administration conditions will vary depending upon factors such as the nature of the protective agent, the condition being treated, the age and size of the animal etc.

Preferably however the immunostimulant of the invention will be administered to the animal in a single pharmaceutical composition with the protective agent.

Suitably the composition will further comprise a pharmaceutically acceptable carrier or diluent. These may be solid or liquid carriers as are known in the art.
Carriers or diluents may vary depending upon the particular nature of the protective agent and the particular polycationic carbohydrate used. They may comprise pharmaceutically acceptable solvents such as water in which the protective agent and the polycationic carbohydrate are dissolved. This type of formulation is particularly suitable when the protective agent is itself water-soluble.

Compositions in the form of solutions of this type suitably contain from 0.1 to 30% w/v and preferably from 1 to 20% w/v of polycationic carbohydrate, depending upon its solubility.

The composition is suitably adapted for parenteral administration such as intramuscular (i.m.) administration, or it may be suitable for administration to mucosal surfaces of the animal.

Administration to mucosal surfaces may be effected by oral application, by pulmonary application, for example by intra-tracheal administration, or particularly by intra-nasal application. In particular, the compositions of the invention are administered by the intra-nasal route.

For many applications however, it has been found preferable that the protective agent and the polycationic carbohydrate immunostimulant is microencapsulated in a polymeric material and thus the carrier is a particulate carrier such as a microsphere or microparticle (also known as a microcapsule), nanocapsule or liposome.

In a preferred embodiment, a polycationic carbohydrate which is able to act as an immunostimulant is provided in the form of a pharmaceutical composition, which composition comprises particles comprising
(i) a biologically active agent which is able to produce an immune response in an animal to which it is administered;
(ii) a first material capable of forming particles; and
(iii) a polycationic carbohydrate according to the present invention.

In a further aspect of the invention there is provided a pharmaceutical composition comprising particles, each particle comprising
(i) a biologically active agent which is able to produce an immune response in an animal to which it is administered;
(ii) a first material capable of forming particles; and
(iii) one or more polycationic carbohydrate which has immunostimulant properties, wherein the polycationic carbohydrate comprises a water-soluble alkylated chitosan derivative or salt thereof and wherein the polycationic carbohydrate is distributed throughout the particle including at the surface.

The particles are suitably incorporated into a composition which is adapted for administration to mucosal surfaces. In addition, the particles may suitably be incorporated into a composition which is adapted for administration using a parenteral route such as i.m. injection.

Suitably the particles comprise microparticles or microspheres or liposomes. The first material used in the compositions of the invention is suitable for forming microspheres or liposomes. Liposome production requires the use of lipids and/or surfactant type molecules as is understood in the art.

Preferably however, the composition of the invention comprises a microsphere. In this case the first material comprises a polymer. It may be a low, medium or high molecular weight polymer. Examples of low molecular weight polymers are polymers which have a molecular weight of between 0.1 and 10kDa, more preferably between 1 and 5 kDa and typically about 2-3kDA.

The use of high molecular weight polymers in the encapsulation of a tetanus vaccine for intramuscular administration has been described (Vaccine 1994, 12, 4, 299-306). A formulation of microencapsulated ricin toxoid vaccine which is applied intranasally has also been described (Vaccine 1994, 14, 11 1031). However, in that case, high molecular weight polymer microparticles (94kDa) were less effective than those prepared from a copolymer of lower molecular weight (72kDa).

The polymeric material used as the first material in the composition of the present invention suitably has a high molecular weight in excess of 94kDa, for example of 100kDa or more.

A particularly suitable polymeric first material for use in the compositions of the invention comprises poly-(L-lactide) or PLA but other high molecular weight polymeric material such as poly(lactic/glycolic acid) PGLA, polycyanacrylates, polyanhydrides or polycaprotactones as are known in the art may be employed.

Suitably the polycationic carbohydrate is present in the composition in an amount of from 0.1% to 10%w/w. Particular examples of suitable polycationic carbohydrates for use in the above described particle compositions include the water-soluble alkylated chitosans present as mixtures thereof with cationic polypeptides, cationic polyamino acids or quaternary ammonium compounds.

The compositions may optionally further comprise agents which stabilise emulsions such as polyvinylalcohol or methyl cellulose.

They will suitably be of an average size of from 0.1µm to 10µm in diameter. Optionally, vaccine compositions may further comprise an additional conventional adjuvant in order to enhance the immune response to the biologically active material administered. Suitable adjuvants include pharmaceutically acceptable adjuvants such as Freund's incomplete adjuvant, aluminium compounds and, preferably adjuvants which are known to up-regulate mucosal responses such as CTB, the non-toxic pentameric B subunit of cholera toxin (CT).

Yet further adjuvants are described and claimed in the applicants copending International application of even date, derived from British Patent Application Nos. 9906694.6 and 9906696.1. In particular these compounds are
A) a polyamino acid,
B) a vitamin or vitamin derivative,
C) cationic pluronics,
D) a clathrate,
E) a complexing agent,
F) cetrimides;
G) an S-layer protein; or
H) methyl-glucamine.

As used herein, the expression "cationic pluronics" includes both pluronics which include cations, as well as those which have been treated such that they are bound to cationic moieties (cationised pluronics).

Suitable biological agents (i) include drugs and therapeutic molecules such as vaccines, antivirals, antibiotics, antifungals, antiparasitics as well as oligonucleotides used in therapies and vaccines.

However in a preferred embodiment, the biologically active agent is an agent that is capable of generating an immune response in an animal to which it is administered and most preferably a protective immune response. Thus the compositions are suitably used as vaccines including those which rely on oligonucleotides or other nucleic acid sequences. In this case, the immunostimulant properties of the compounds A-H are used.

Suitably the said adjuvant chemical is soluble in water. Suitably the composition is suitable for non-parenteral administration for example to mucosal surfaces or for topical application to the skin. Particularly preferred compositions are suitable for administration to mucosal surfaces.

Alternatively, the composition is suitable for parenteral administration for example by intramuscular (i.m.) administration.

Administration to mucosal surfaces may be effected by oral application, by pulmonary application, for example by intratracheal administration, or particularly by intra-nasal application. In particular, the compositions of the invention are administered by the intra-nasal route.

Examples of adjuvant chemicals in category (A) above include polyamino acids such poly-ornithine, for example of molecular weight from 5 to 150kDa.

Particular examples of adjuvant chemicals in category (B) above are vitamins or vitamin derivatives such as vitamin E or derivatives for example vitamin E TPGS (d-alpha tocopheryl polyethylene glycol 1000 succinate).

Particular cationic pluronics in category (C) above, are block copolymers or surfactants which are positively charged, in particular with NH₂⁺ groups. These are available commercially for example from ICI Ltd (UK) sold under the trade names P101 and P121.

Examples of clathrates in category (D) above include in particular cyclodextrins and their derivatives such as dimethyl β cyclodextrin.

Suitable complexing agents in category (E) above are bile salts, in particular those which form complexes with fatty acids such as deoxycholic acid.

Examples of cetrimides in category (F) are quaternary ammonium compounds used as preservatives.

In particular, the compositions of the invention may further comprise a cationic pluronic as described above. These may be included either within the particles, or as a component of the dosing mixture or both. In a particularly preferred embodiment, the cationic pluronic is formed into microparticles and in particular nanospheres, which are then coated with the polycationic carbohydrate such as chitosan. Biologically active material may then be absorbed onto the coated particles.

According to a further aspect of the invention there is provided a pharmaceutical composition comprising particles, each particle comprising
(i) a biologically active agent which is able to produce an immune response in an animal to which it is administered;
(ii) a cationic pluronic; and
(iii) one or more polycationic carbohydrate which has immunostimulant properties, wherein the polycationic carbohydrate comprises a water-soluble alkylated chitosan derivative or salt thereof and the particles comprise nanospheres of the cationic pluronic which are surface modified with the polycationic carbohydrate.

The compositions may also comprise other known composition components such as colouring agents and preservatives and in particular cetrimide. These are suitably present in amounts of from 0.1 to 0.7%w/v. In a particular embodiment, the microspheres or liposomes used in the compositions may further comprise S-layer proteins, in particular, S-layer proteins derived from bacteria against which the biologically active agent produces a protective immune response. These may be distributed throughout the microspheres or liposomes but are preferably coated on the surface. It has been shown (Sleyr et al., Crystalline bacterial cell surface proteins. Biotechnology Intelligence Unit, 1996, R.G. Landes Company and Academic Press Inc.) that the stability of liposomes can be increased by such coatings. S-layer proteins are found on the surface of most bacteria and form a regular two dimensional array known as an S-layer. Isolated S-layer proteins are able to form entropy driven monomolecular arrays in suspension, and on the surface of structures such as liposomes.

The above-described particle composition is particularly suitable for intranasal application. They may comprise particles such as microparticles per se which are optionally preserved, for example by lyophilisation, or the microparticles may be combined with a pharmaceutically acceptable carrier or excipient. Examples of suitable carriers include solid or liquid carriers as is understood in the art.

The invention further provides a method of producing a pharmaceutical therapeutic vaccine, which method comprises encapsulating a biologically active agent as described above in a first polymeric material which has a high molecular weight and in particular a molecular weight of 100kDa or more, in the presence of the polycationic carbohydrate. The polycationic carbohydrate may be incorporated within the microparticle, or at the surface, or preferably is distributed throughout the microparticle including at the surface.

In some cases, it may preferably be adsorbed onto the surface of a pre-formed particle. It is possible also to load biologically active agent (such as a protective antigen) onto the thus formed particles.

Methods of forming liposomes are well known in the art. They include dispersion of dehydrated lipid films into aqueous media, emulsion techniques and lyophilisation methods as are well known in the art.

According to a yet further aspect of the present invention there is provided a method for producing a pharmaceutical composition, which method comprises forming an emulsion of a biologically active agent and a first polymeric material, in the presence of the immunostimulant polycationic carbohydrate, and dropping the resultant emulsion into a secondary aqueous phase which also contains the immunostimulant polycationic carbohydrate.

Microparticles of the invention are suitably prepared using a double emulsion solvent evaporation method. Briefly, the biologically active agent, suitably in a lyophilised state, is suspended in an aqueous solution of the first polymer such as polyvinyl alcohol (PVA) and the polycationic carbohydrate. A solution of the high molecular weight polymer in an organic solvent such as dichloromethane, is added with vigorous mixing. The resultant emulsion is then dropped into a secondary aqueous phase, optionally containing polycationic carbohydrate, with vigorous stirring. After addition, the organic solvent is allowed to evaporate off and the resultant microspheres separated.

Preferred biologically active agents and first materials and polycationic carbohydrates are as described above.

The compositions will suitably comprise an appropriate dosage unit of the active agent. This will vary depending upon the nature of the active agent being employed, the nature of the patient, the condition being treated and other clinical factors.

In general however, the composition of the invention will comprise approximately 2 to 10 wt% of active ingredient.

In microparticle containing compositions of the invention the amount of first material, in particular the high molecular weight polymer, in the composition will be of the order of 70 to 99wt% of the composition, and suitably from 90 to 99wt% of the polymer components will be the first polymer. The amount of polycationic carbohydrate material, such as water-soluble alkylated chitosan or a mixture of water-soluble alkylated chitosan with other positively charged molecules, will be of the order of 0.1 to 10 wt % of the composition.

In use, a reasonable dosage for nasal administration would be of the order of 0.05g.

The invention is of use in protecting a mammal against infection, by the administration of a vaccine composition as described above to a mucosal surface, in particular a nasal surface, of a mammal.

The applicants have demonstrated that it is possible to protect experimental animals from inhalation challenge with *Y*. *pestis* through i.n. administration of a combined sub-unit vaccine. The adjuvantisation of these sub-units is advantageous in enhancing the immune response as is microencapsulation of the sub-units. The high molecular weight polymer utilised in the compositions of the invention appears to be particularly well suited to intra-nasal delivery.

Alternatively, however, the immunostimulants of the invention may be administered as i.m. formulations and long term beneficial effects are still noted.

The invention will now be particularly described by way of example with reference to the accompanying drawings in which:
Figure 1 illustrates the serum immune response in mice to nasally delivered microencapsulated and free diptheria toxoid with 10 lf units on day 1 and day 67, where the first column represents results with microsphere without chitosan, the second column represents the results of microspheres with chitosan and the third column shows the results with free diptheria toxoid alone;
Figure 2 illustrates the specific serum antibody responses following a single nasal application of 1µg V and 5µg F1 antigens of *Yersinia pestis* in compositions according to the invention; and
Figure 3 shows the specific serum antibody responses over a period up to 86 days following intramuscular injection of free of microencapsulated BSA in the presence or absence of chitosan;
Figure 4 shows the serum immune response to nasally delivered Diptheria toxoid (DT) adsorbed onto nanoparticles whose surface has been modified cationic with chitosan derivatives;
Figure 5 shows the results of photon correlation spectroscopy (PCS) on particles produced for use in the invention, showing that the number mean diameter (dₙ) and the volume mean diameter (dᵥ) were both around 150nm; and
Figure 6 illustrates the immune response to Diptheria toxoid (DT) in various formulations applied by i.m. routes, where P101 is a pluronic 101 and P121 is a block co-polymer available from ICI Ltd, UK.

### Example 1

### Microencapsulation of diptheria toxoid

Poly-L-lactide of molecular weight 100kDa (Polysciences Inc. USA) was used in a modification of the double emulsion solvent evaporation method (Y. Ogawa et al., Chem. Pharm. Bull., 36 (1988) 1095-1103). Briefly, 1.5ml of a 0.75% w/v of chitosan solution containing diptheria toxoid was vigorously mixed with 200mg of 100K PLA polymer dissolved in 5ml of HPLC grade dichloromethane (DCM) using a Silverson homogeniser (Silverson, UK) for 1 minute. The resultant primary emulsion was added, drop by drop, into a secondary aqueous phase (75ml) containing 0.5%w/v chitosan and homogenised using a Silverson homogeniser for 5 minutes. This secondary phase was gently stirred overnight until the dichloromethane had evaporated. Microspheres were recovered by centrifugation, washed with double distilled water three times and then lyophilised.

### Example 2

### Immunisation Study

Balb/c female mice (25g, 6-week old) were lightly anaesthetised using an inhaled gaseous mixture of 3% halomethane (RMB Animal Health Ltd., UK) in oxygen (300cm³ min⁻¹) and nitrous oxide (100cm³min⁻¹) for intranasal dosing procedures. Groups of mice received one of the following treatments:
(1) Microspheres prepared as described in Example 1 but in the absence of chitosan;
(2) Microspheres prepared as described in Example 1; and
(3) Free diptheria toxoid solution.

Each were administered in 50µl of PBS using a micropipette. Groups of mice each received 10 lf units on day 1 and day 67 of either microencapsulated or free diptheria toxoid.
Serum immune responses were monitored. Tail vein blood samples were taken from all animals on days 14, 28, 95 and 151 of the experiment. Titration of IgG and IgA antibody isotypes in serum samples was achieved using an ELISA. Briefly, individual serum samples were aliquoted to microtitre plates pre-coated with diptheria toxoid. Binding of serum antibody was detected with peroxidase-labelled secondary antibody to mouse IgG (Sigma A4416) or AgA (Sigma A4789). Antibody titre was estimated as the maximum dilution of the serum giving an absorbance reading greater that the maximum optical density (OD) of titrated naive serum. From this, mean titres ± standard deviation (SD) were derived per treatment group.

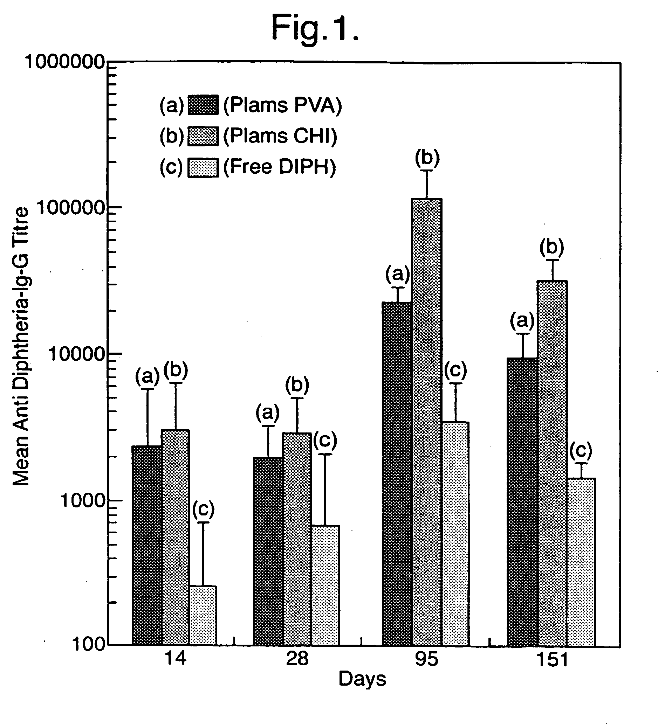

The results are shown in Figure 1. Throughout the 151 day schedule, mice dosed with microencapsulated antigen in the absence of chitosan (Group 1) maintained statistically elevated serum IgG titres to diptheria toxoid in comparison to animals treated with free vaccine (Group 3) (Figure 1). However, the levels of IgG titres to diptheria toxoid in Group 2 animals was consistently higher still indicating that the presence of chitosan in the microparticle enhances the immune response to the toxoid.

### Example 3

### Use of absorption enhancers to enhance immunological response to intranasally administered subunit vaccines

Five groups of five (n=5) BALB/c mice were intranasally immunised with admixed F1 (5 µg) and V (1 µg). The five treatment groups received the subunits in conjunction with either: 1) Phosphate buffered saline (pH 7.4); 2) 0.2% w/v chitosan HCL; 3) 0.2% w/v TMC 60; (4) 0.2% w/v TMC 40; (5) 0.2% w/v TMC 20. A further group of animals acted as a control.

Mice were lightly anaesthetised with an inhaled gaseous mixture of 3% (v/v) halothane (RMB Animal Health Ltd., UK) in oxygen (300cm³ min⁻¹) and nitrous oxide (100cm³ min⁻¹) for i.n. dosing procedures. Each mouse received a 15 µl volume of liquid administered with a micropipette. Tail vein blood samples were taken on day 14, and serum was analysed for the presence of anti-V and anti-F1 IgG antibodies using an indirect ELISA protocol (Eyles, J. E. et al. Vaccine (1998) 16:698-707).

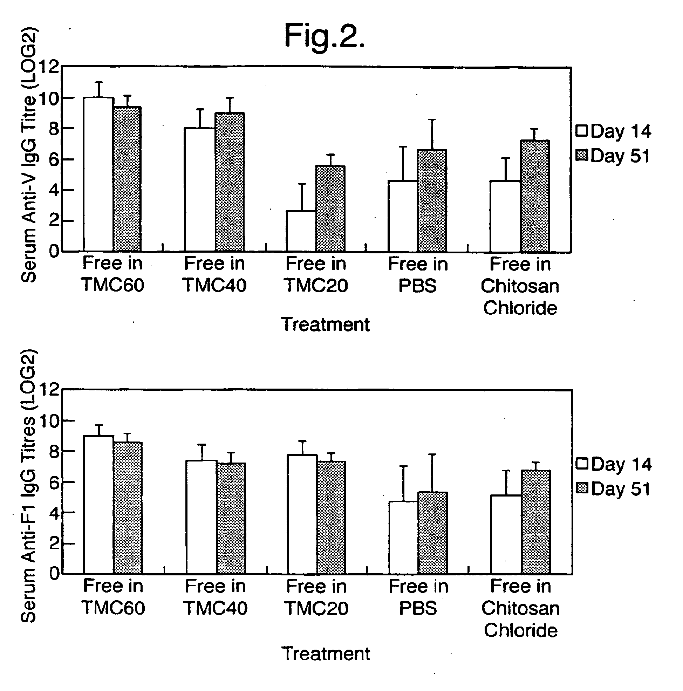

The results (Figure 2) indicate that mucosal co-administration of TMC60 or TMC40 augments the humoral response to F1 and V above and beyond that generated by i.n. instillation of F1 and V in phosphate buffered saline or chitosan HCl. TMC20 failed to improve titre to V, although the effect on immunity to F1 was comparable with that of the more substituted chitosan derivatives (TMC40 & 60).

### Example 4

### Intramuscular administration of immunostimulants.

Microspheres incorporating bovine serum albumin (BSA) were prepared from poly-L-lactic acid of molecular weights 2kD, 50kD or 100kD (or a combination of these) in the presence or absence of chitosans using a double emulsion method (dichloromethane/water). These preparations were administered to mice in a single intramuscular dose in a 50µl volume of sterile PBS ('Day 0' of the study, 15µg BSA equivalent, typically 1mg microspheres). Blood samples were taken from mice at a number of subsequent timepoints and the serum analysed by ELISA for anti-BSA IgG levels.

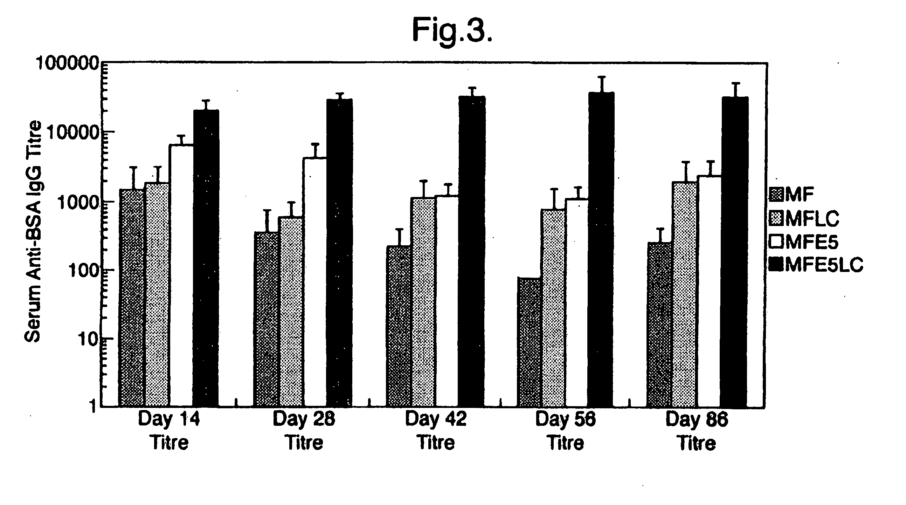

The results are shown in Figure 3. In this graph, the following key applies:
M= intramuscular;
F = free BSA;
E5 = BSA encapsulated into microspheres composed from a 1:1 mass ratio of 2kD:100kD PLA;
LC = low MW chitosan.

Surprisingly it was found that free antigen plus microencapsulated BSA in the presence of chitosan performed better in vivo than other dosing administrations containing only one of these components with free antigen, or free antigen administered alone.

### Example 5

### surface modification using chitosans

500mg of PDLA (124kD) was dissolved in dichloromethane (DCM). To this solution, an aqueous solution of 2.5%(w/v) polyvinylalcohol (PVA) was added while probe-sonicated. The resultant primary emulsion was added to 20ml of a colled aqueous solution of 1.5% (w/v) PVA under homogenisation (Silverson SL-2 homogeniser), to form a secondary emulsion. A further 10ml of water was added to the resulting emulsion and the formulation stirred overnight. Particles were washed and collected by centrifugation with distilled water at 3x20 min at 15,000rpm, before finally being freeze-dried.

Four batches of particles were prepared as described above, three of which were surface-modifed, either with N-carboxymethyl chitosan (Canada), chitosan chloride or chitosan glutamate (Pronovo, Norway) by adding 10mg of the relevant chitosan to 90mg of particles suspended in 1 ml of water. This mixture was placed in a bath-sonicator for 5 minutes before being shaken for one hour and finally freeze-dried. The fourth batch remained unmodified.

Saline solution (0.5ml) containing 100Lf diptheria toxoid was added to 10mg of the particles. Resulting suspensions were shaken overnight to enable adsorption of the antigen to the particle surfaces. following incubation, particles were washed by centrifugation with distilled water, as described above. In all cases, the antigen loading was determined to be in the region of 0.5% (w/w), giving an encapsulation efficiency of generally 50-60%.

Particles were fully characterised and mean particle diameters were found to be around 340nm for all batches.

Groups of Balb/c mice (n=5 per group) were dosed intranasally with the four freshly prepared suspensions, with doses equivalent to 5Lf units of DT per mouse, and a dosing volume of 20µl. Animals were bled periodically and ELISAs carried out to determine serum levels of anti-DT specific IgG titres.

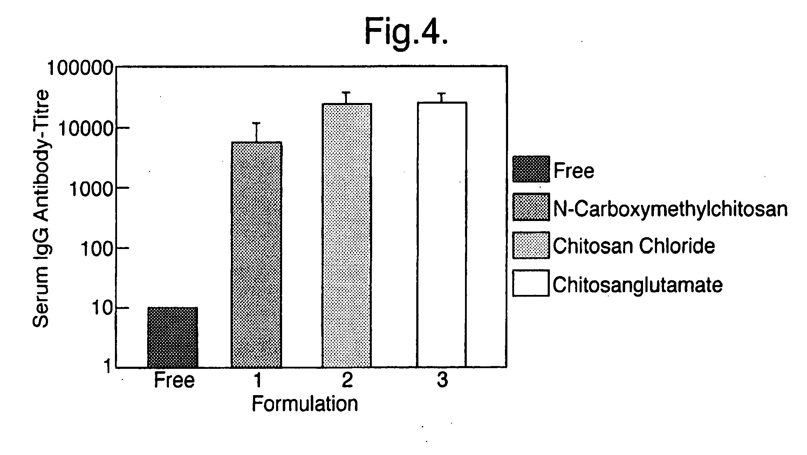

The results for blood collected on Day 28 are shown in Figure 4.

### Example 6

### Immune responses to intramuscularly delivered diptheria toxoid (DT)

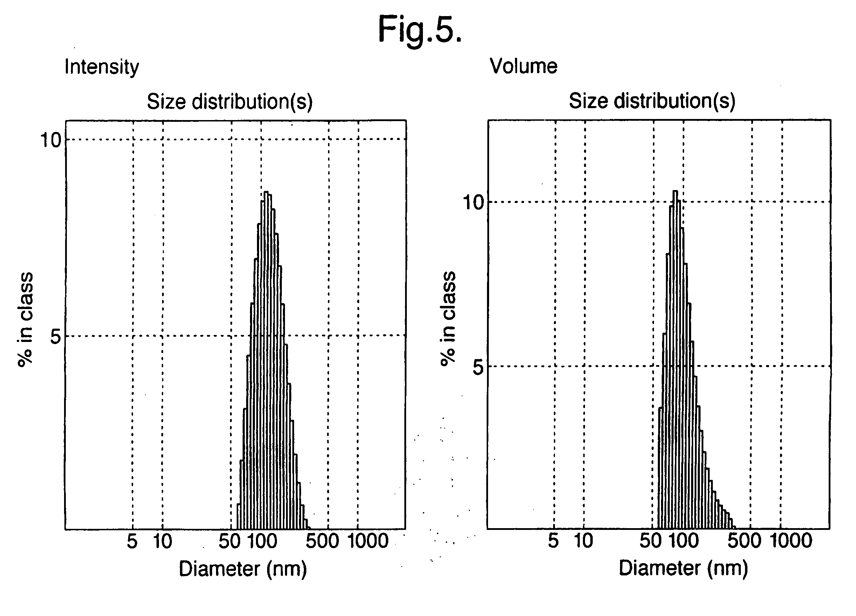

Eight formulations of pluronic/chitosan nanoparticles were prepared, by a simple sonication method. Deacetylated high molecular weight chitosan was obtained from Fluka. Three process variables were : type of Pluronic used (P101, P121 obtained from ICI Limited, UK), volume of pluronic added (75µl or 200µl per 2ml water), and addition or omission of chitosan. Briefly, to 2ml of double-distilled water, a small volume (75µl or 200µl) of the appropriate pluronic liquid (P101 or P121) was added. Mixtures were vortexed for one minute and sonicated for a further one minute. In order to coating the pluronic particles with chitosan, 100µl of a solution of 0.1% w/v high molecular weight chitosan in 2% w/v glacial acetic acid was added to 100µl of each formulation. Finally, the diphtheria toxoid (DT) was adsorbed to the 200µl of coated and non-coated particles by the addition of 12µl of a solution of DT in water (4450lf units per ml). This preparation is a colloidal dispersion in water with a mean particle diameter of generally between 100-600nm. An example of a typical photon correlation spectroscopy (PCS) printout is shown in Figure 5.

Following characterisation of the prepared particulate formulations, groups of four or five female Balb/c mice were given a single dose of 50µl intramuscularly. The total equivocal dose for each animal was 5Lf units of DT. The final concentration of pluronics in the dosing medium was 5% (v/v) and chitosan 0.05% (w/v). Animals were bled periodically and ELISAs carried out to determine serum levels of anti-DT specific IgG titres.

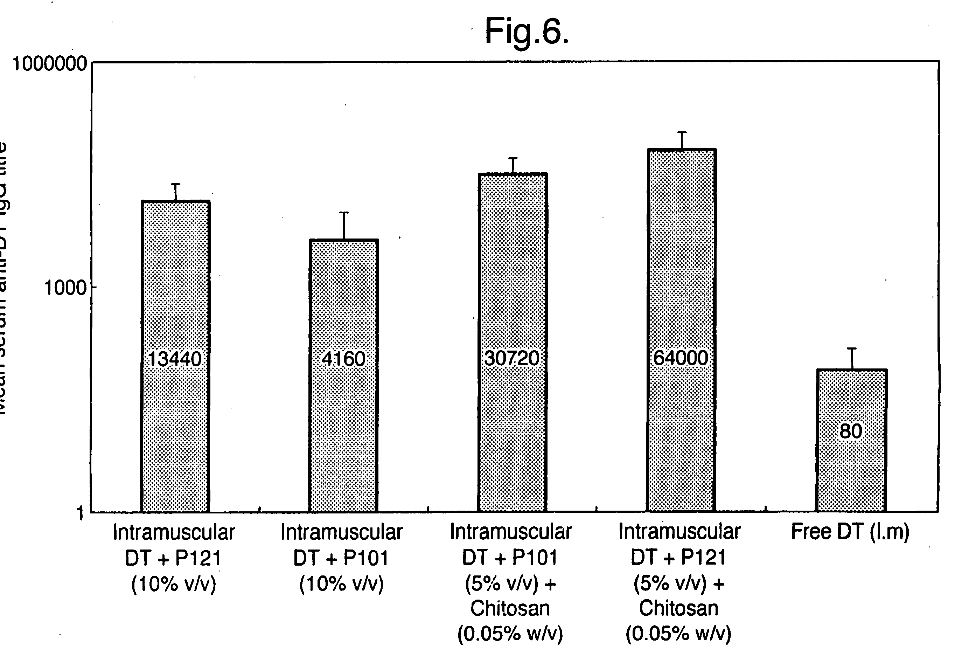

Mean serum anti-DT IgG titres are shown herewith in Figure 6. It is clear from these results, that pluronics produce an enhanced immune response, either alone or in combination with chitosan.

## Claims

1. The use of a polycationic carbohydrate as an immunostimulant in the manufacture of a vaccine for protecting an animal against infection by a pathogen, wherein the polycationic carbohydrate comprises a water-soluble alkylated chitosan derivative or salt thereof.

2. The use according to claim 1 in which the water-soluble alkylated chitosan is selected from trimethyl chitosan chloride, N-carboxymethyl chitosan or trimethylchitosan.

3. The use according to claim 1 or 2, wherein the polycationic carbohydrate is present as a mixture thereof with a cationic polypeptide, cationic polyamino acid or a quaternary ammonium compound.

4. A pharmaceutical composition comprising a biologically active agent, which is capable of generating a protective immune response in an animal, and a polycationic carbohydrate, wherein the polycationic carbohydrate comprises a water-soluble alkylated chitosan derivative or salt thereof.

5. A pharmaceutical composition according to claim 4, in which the water-soluble alkylated chitosan is selected from trimethyl chitosan chloride, N-carboxymethyl chitosan or trimethylchitosan.

6. A pharmaceutical composition according to claim 4 or 5, wherein the polycationic carbohydrate is present as a mixture thereof with a cationic polypeptide, cationic polyamino acid or a quaternary ammonium compound.

7. A pharmaceutical composition according to claim 4, 5 or 6 which further comprises a diluent or carrier.

8. A pharmaceutical composition, which comprises particles comprising:
(i) a biologically active agent which is able to produce an immune response in an animal to which it is administered;
(ii) a first material capable of forming particles; and
(iii) a polycationic carbohydrate wherein the polycationic carbohydrate comprises a water-soluble alkylated chitosan derivative or salt thereof.

9. A pharmaceutical composition comprising particles, each particle comprising:
(i) a biologically active agent which is able to produce an immune response in an animal to which it is administered;
(ii) a first material capable of forming particles; and
(iii) one or more polycationic carbohydrate which has immunostimulant properties, wherein the polycationic carbohydrate comprises a water-soluble alkylated chitosan derivative or salt thereof and wherein the polycationic carbohydrate is distributed throughout the particle including at the surface.

10. A pharmaceutical composition according to any one of claims 4 to 9 wherein the polycationic carbohydrate is present as a mixture thereof with a cationic polypeptide, a cationic polyamino acid or a quaternary ammonium compound.

11. A composition according to any one of claims 4 to 10 wherein the water-soluble alkylated chitosan is selected from trimethyl chitosan, trimethyl chitosan chloride or N-carboxymethyl chitosan.

12. A composition according to any one of claims 8 to 11 wherein the particle comprises microspheres, microparticles or liposomes.

13. A composition according to claim 12 wherein the particle comprises a microparticle.

14. A composition according to any one of claims 8 to 13 wherein the first material is a polymeric material which has a molecular weight of 100kDa or more.

15. A composition according to any one of claims 8 to 14 wherein the first material comprises poly-(L-lactide).

16. A composition according to any one of claims 8 to 15 wherein the ratio of the first material to the polycationic carbohydrate is from 99:1 to 9:1 w/w.

17. A composition according to any one of claims 8 to 16 wherein the biologically active agent is capable of generating a protective immune response against tetanus, diptheria, or *Yersinia pestis.*

18. A composition according to claim 17 wherein the biologically active agent comprises a combination of the V antigen of *Y. pestis* or an immunologically active fragment thereof, and the F1 antigen of *Y*. *pestis* or an immunologically active fragment thereof.

19. A composition according to any one of claims 8 to 18 which is adapted for intranasal application.

20. A composition according to any one of claims 8 to 18 which is adapted for parenteral administration.

21. A composition according to any one of claims 4 to 20 which further comprises a chemical compound selected from
A) a polyamino acid,
B) a vitamin or vitamin derivative,
C) cationic pluronics,
D) a clathrate,
E) a complexing agent,
F) cetrimides;
G) an S-layer protein; or
H) methyl-glucamine.

22. A composition according to claim 21 wherein the further compound is a cationic pluronic and the composition comprises nanospheres of a cationic pluronic which are surface modified with chitosan.

23. A pharmaceutical composition comprising particles, each particle comprising
(i) a biologically active agent which is able to produce an immune response in an animal to which it is administered;
(ii) a cationic pluronic; and
(iii) one or more polycationic carbohydrate which has immunostimulant properties, wherein the polycationic carbohydrate comprises a water-soluble alkylated chitosan derivative or salt thereof and wherein the particles comprise nanospheres of the cationic pluronic which are surface modified with the polycationic carbohydrate.

24. A pharmaceutical composition according to claim 23 wherein the polycationic carbohydrate is present as a mixture thereof with a cationic polypeptide, a cationic polyamino acid or a quaternary ammonium compound.

25. A composition according to claim 24 wherein the water-soluble alkylated chitosan is selected from trimethyl chitosan, trimethyl chitosan chloride or N-carboxymethyl chitosan.

26. A method for producing a pharmaceutical composition according to claim 8 or 9 and to any one of claims 10 to 21 when dependant on claim 8 or 9, which method comprises encapsulating the biologically active agent in the first material, in the presence of the polycationic carbohydrate.

27. A method for producing a pharmaceutical composition according to claim 8 or 9 and to any one of claims 10 to 21 when dependant on claim 8 or 9, which method comprises forming an emulsion of the biologically active agent and the first material, in the presence of the polycationic carbohydrate, and dropping the resultant emulsion into a secondary aqueous phase which also contains the polycationic carbohydrate.

28. A method for producing a pharmaceutical composition according to claim 23, which method comprises forming a nanosphere of the cationic pluronic, depositing a layer of the polycationic carbohydrate thereon, and thereafter adsorbing the biologically active agent.

29. A water-soluble alkylated chitosan for use as an immunostimulant in the treatment of infection by a pathogen.

## Patentansprüche

1. Verwendung eines polykationischen Kohlenhydrats als Immunstimulans bei der Herstellung eines Impfstoffs zum Schutz eines Lebewesens gegen Infektion durch ein Pathogen, wobei das polykationische Kohlenhydrat ein wasserlösliches alkyliertes Chitosanderivat oder ein Salz davon ist.

2. Verwendung nach Anspruch 1, wobei das wasserlösliche alkylierte Chitosan unter Trimethylchitosanchlorid, N-Carboxymethylchitosan oder Trimethylchitosan ausgewählt ist.

3. Verwendung nach Anspruch 1 oder 2, wobei das polykationische Kohlenhydrat im Gemisch mit einem kationischen Polypeptid, einer kationischen Polyaminosäure oder einer quaternären Ammoniumverbindung vorliegt.

4. Pharmazeutische Zusammensetzung, die ein biologisch wirksames Mittel, das befähigt ist, eine schützende Immunantwort in einem Lebewesen zu erzeugen, und ein polykationisches Kohlenhydrat enthält, wobei das polykationische Kohlenhydrat ein wasserlösliches alkyliertes Chitosanderivat oder ein Salz davon ist.

5. Pharmazeutische Zusammensetzung nach Anspruch 4, bei der das wasserlösliche alkylierte Chitosan unter Trimethylchitosanchlorid, N-Carboxymethylchitosan oder Trimethylchitosan ausgewählt ist.

6. Pharmazeutische Zusammensetzung nach Anspruch 4 oder 5, in der das polykationische Kohlenhydrat im Gemisch mit einem kationischen Polypeptid, einer kationischen Polyaminosäure oder einer quaternären Ammoniumverbindung vorliegt.

7. Pharmazeutische Zusammensetzung nach Anspruch 4, 5 oder 6, die ferner ein Verdünnungsmittel oder einen Träger enthält.

8. Pharmazeutische Zusammensetzung, die aus Partikeln besteht und aufweist:
(i) ein biologisch wirksames Mittel, das befähigt ist, eine Immunantwort in einem Lebewesen, dem es verabreicht wird, zu erzeugen,
(ii) ein erstes Material, das zur Bildung von Partikeln befähigt ist, und
(iii) ein polykationisches Kohlenhydrat, wobei das polykationische Kohlenhydrat ein wasserlösliches alkyliertes Chitosanderivat oder ein Salz davon ist.

9. Pharmazeutische Zusammensetzung, die aus Partikeln besteht, wobei jedes Partikel enthält:
(i) ein biologisch wirksames Mittel, das befähigt ist, eine Immunantwort in einem Lebewesen, dem es verabreicht wird, zu erzeugen,
(ii) ein erstes Material, das zur Bildung von Partikeln befähigt ist, und
(iii) ein oder mehrere polykationische Kohlenhydrate mit Immunstimulationseigenschaften, wobei das polykationische Kohlenhydrat ein wasserlösliches alkyliertes Chitosanderivat oder ein Salz davon ist und das polykationische Kohlenhydrat über das Partikel sowie auf seiner Oberfläche verteilt ist.

10. Pharmazeutische Zusammensetzung nach einem der Ansprüche 4 bis 9, bei der das polykationische Kohlenhydrat im Gemisch mit einem kationischen Polypeptid, einer kationischen Polyaminosäure oder einer quaternären Ammoniumverbindung vorliegt.

11. Zusammensetzung nach einem der Ansprüche 4 bis 10, bei der das wasserlösliche alkylierte Chitosan unter Trimethylchitosan, Trimethylchitosanchlorid und N-Carboxymethylchitosan ausgewählt ist.

12. Zusammensetzung nach einem der Ansprüche 8 bis 11, wobei die Partikel Mikrokügelchen, Mikropartikel oder Liposomen sind.

13. Zusammensetzung nach Anspruch 12, bei der die Partikel Mikropartikel sind.

14. Zusammensetzung nach einem der Ansprüche 8 bis 13, bei der das erste Material ein Polymermaterial ist, das ein Molekulargewicht von 100 kDa oder mehr aufweist.

15. Zusammensetzung nach einem der Ansprüche 8 bis 14, bei der das erste Material Poly-(L-lactid) ist.

16. Zusammensetzung nach einem der Ansprüche 8 bis 15, bei der das Verhältnis von erstem Material zu polykationischem Kohlenhydrat im Bereich 99 : 1 bis 9 : 1 (G/G) liegt.

17. Zusammensetzung nach einem der Ansprüche 8 bis 16, bei der das biologisch wirksame Mittel befähigt ist, eine schützende Immunantwort gegen Tetanus, Diphtherie oder *Yersinia pestis* zu erzeugen.

18. Zusammensetzung nach Anspruch 17, bei der das biologisch wirksame Mittel eine Kombination des V-Antigens von *Y. pestis* oder eines immunologisch aktiven Fragments davon mit dem F1-Antigen von *Y. pestis* oder einem immunologisch aktiven Fragment davon ist.

19. Zusammensetzung nach einem der Ansprüche 8 bis 18, die zur intranasalen Anwendung hergerichtet ist.

20. Zusammensetzung nach einem der Ansprüche 8 bis 18, die zur parenteralen Verabreichung hergerichtet ist.

21. Zusammensetzung nach einem der Ansprüche 4 bis 20, die ferner eine chemische Verbindung enthält, die ausgewählt ist unter
A) einer Polyaminosäure,
B) einem Vitamin oder Vitaminderivat,
C) kationischen Pluronics,
D) einem Clathrat,
E) einem Komplexbildner,
F) Cetrimiden,
G) einem S-Layer-Protein oder
H) Methylglucamin.

22. Zusammensetzung nach Anspruch 21, bei der die weitere Verbindung ein kationisches Pluronic ist und die Zusammensetzung Nanokügelchen eines kationischen Pluronics enthält, die mit Chitosan oberflächenmodifiziert sind.

23. Pharmazeutische Zusammensetzung, die aus Partikeln besteht, wobei jedes Partikel aufweist:
(i) ein biologisch wirksames Mittel, das befähigt ist, eine Immunantwort in einem Lebewesen, dem es verabreicht wird, zu erzeugen,
(ii) ein kationisches Pluronic und
(iii) ein oder mehrere polykationische Kohlenhydrate mit Immunstimulationseigenschaften, wobei das polykationische Kohlenhydrat ein wasserlösliches alkyliertes Chitosanderivat oder ein Salz davon ist und wobei die Partikel Nanokügelchen des kationischen Pluronics sind, die mit dem polykationischen Kohlenhydrat oberflächenmodifiziert sind.

24. Pharmazeutische Zusammensetzung nach Anspruch 23, bei der das polykationische Kohlenhydrat im Gemisch mit einem kationischen Polypeptid, einer kationischen Polyaminosäure oder einer quaternären Ammoniumverbindung vorliegt.

25. Zusammensetzung nach Anspruch 24, bei der das wasserlösliche alkylierte Chitosan unter Trimethylchitosan, Trimethylchitosanchlorid oder N-Carboxymethylchitosan ausgewählt ist.

26. Verfahren zur Herstellung einer pharmazeutischen Zusammensetzung nach Anspruch 8 oder 9 und einem der Ansprüche 10 bis 21 in Abhängigkeit von Anspruch 8 oder 9, das die Einkapselung des biologisch wirksamen Mittels im ersten Material in Gegenwart des polykationischen Kohlenhydrats umfaßt.

27. Verfahren zur Herstellung einer pharmazeutischen Zusammensetzung nach Anspruch 8 oder 9 und einem der Ansprüche 10 bis 21 in Abhängigkeit von Anspruch 8 oder 9, das umfaßt:
Erzeugen einer Emulsion des biologisch wirksamen Mittels und des ersten Materials in Gegenwart des polykationischen Kohlenhydrats und
Eintropfen der resultierenden Emulsion in eine zweite wässerige Phase, die ebenfalls das polykationische Kohlenhydrat enthält.

28. Verfahren zur Herstellung einer pharmazeutischen Zusammensetzung nach Anspruch 23, das umfaßt: Erzeugung eines Nanokügelchens des kationischen Pluronics, Abscheidung einer Schicht des polykationischen Kohlenhydrats darauf und anschließend Adsorbieren des biologisch wirksamen Mittels.

29. Wasserlösliches alkyliertes Chitosan zur Verwendung als Immunstimulans bei der Behandlung gegen Infektion durch ein Pathogen.

## Revendications

1. Utilisation d'un hydrate de carbone polycationique en tant qu'immunostimulant dans la fabrication d'un vaccin pour protéger un animal contre une infection par un pathogène, dans laquelle l'hydrate de carbone polycationique comprend un dérivé de chitosane alkylé hydrosoluble ou un sel de celui-ci.

2. Utilisation selon la revendication 1, dans laquelle le chitosane alkylé hydrosoluble est choisi parmi le chlorure de triméthylchitosane, le N-carboxyméthylchitosane ou le triméthylchitosane.

3. Utilisation selon la revendication 1 ou 2, dans laquelle l'hydrate de carbone polycationique est présent sous forme d'un mélange de celui-ci avec un polypeptide cationique, un acide polyaminé cationique ou un composé d'ammonium quaternaire.

4. Composition pharmaceutique comprenant un agent biologiquement actif, qui est capable de générer une réponse immunitaire protectrice chez un animal, et un hydrate de carbone polycationique, dans laquelle l'hydrate de carbone polycationique comprend un dérivé de chitosane alkylé hydrosoluble ou un sel de celui-ci.

5. Composition pharmaceutique selon la revendication 4, dans laquelle le chitosane alkylé hydrosoluble est choisi parmi le chlorure de triméthylchitosane, le N-carboxyméthylchitosane ou le triméthylchitosane.

6. Composition pharmaceutique selon la revendication 4 ou 5, dans laquelle l'hydrate de carbone polycationique est présent sous forme d'un mélange de celui-ci avec un polypeptide cationique, un acide polyaminé cationique ou un composé d'ammonium quaternaire.

7. Composition pharmaceutique selon la revendication 4, qui comprend en outre un diluant ou support.

8. Composition pharmaceutique, qui comprend des particules comprenant :
(i) un agent biologiquement actif qui est capable de produire une réponse immunitaire chez un animal auquel il est administré ;
(ii) un premier matériau capable de former des particules ; et
(iii) un hydrate de carbone polycationique, dans lequel l'hydrate de carbone polycationique comprend un dérivé de chitosane alkylé hydrosoluble ou un sel de celui-ci.

9. Composition pharmaceutique comprenant des particules, chaque particule comprenant :
(i) un agent biologiquement actif qui est capable de produire une réponse immunitaire chez un animal auquel il est administré ;
(ii) un premier matériau capable de former des particules ; et
(iii) un ou plusieurs hydrates de carbone polycationiques qui possèdent des propriétés immunostimulantes, dans laquelle l'hydrate de carbone polycationique comprend un dérivé de chitosane alkylé hydrosoluble ou un sel de celui-ci et dans laquelle l'hydrate de carbone polycationique est réparti dans l'ensemble de la particule y compris à la surface.

10. Composition pharmaceutique selon l'une quelconque des revendications 4 à 9, dans laquelle l'hydrate de carbone polycationique est présent sous forme d'un mélange de celui-ci avec un polypeptide cationique, un acide polyaminé cationique ou un composé d'ammonium quaternaire.

11. Composition selon l'une quelconque des revendications 4 à 10, dans laquelle le chitosane alkylé hydrosoluble est choisi parmi le triméthylchitosane, le chlorure de triméthylchitosane ou le N-carboxyméthylchitosane.

12. Composition selon l'une quelconque des revendications 8 à 11, dans laquelle la particule comprend des microsphères, des microparticules ou des liposomes.

13. Composition selon la revendication 12, dans laquelle la particule comprend une microparticule.

14. Composition selon l'une quelconque des revendications 8 à 13, dans laquelle le premier matériau est un matériau polymère qui a un poids moléculaire de 100 kDa ou plus.

15. Composition selon l'une quelconque des revendications 8 à 14, dans laquelle le premier matériau comprend du poly-(L-lactide).

16. Composition selon l'une quelconque des revendications 8 à 15, dans laquelle le rapport du premier matériau sur l'hydrate de carbone polycationique est de 99/1 à 9/1 p/p.

17. Composition selon l'une quelconque des revendications 8 à 16, dans laquelle l'agent biologiquement actif est capable de générer une réponse immunitaire protectrice contre le tétanos, la diphtérie, ou *Yersinia pestis.*

18. Composition selon la revendication 17, dans laquelle l'agent biologiquement actif comprend une combinaison de l'antigène V de *Y. pestis* ou d'un fragment immunologiquement actif de celui-ci, et de l'antigène F1 de *Y. pestis* ou d'un fragment immunologiquement actif de celui-ci.

19. Composition selon l'une quelconque des revendications 8 à 18, qui est adaptée pour une application intranasale.

20. Composition selon l'une quelconque des revendications 8 à 18, qui est adaptée pour une administration parentérale.

21. Composition selon l'une quelconque des revendications 4 à 20, qui comprend en outre un composé chimique choisi parmi :
A) un acide polyaminé,
B) une vitamine ou un dérivé de vitamine.
C) un composé Pluronic^{®} cationique,
D) un clathrate,
E) un agent complexant,
F) un cétrimonium,
G) une protéine de couche S ; ou
H) une méthyl-glucamine.

22. Composition selon la revendication 21, dans laquelle ledit autre composé est un composé Pluronic^{®} cationique et la composition comprend des nanosphères d'un composé Pluronic^{®} cationique qui sont modifiées en surface avec du chitosane.

23. Composition pharmaceutique comprenant des particules, chaque particule comprenant :
(i) un agent biologiquement actif qui est capable de produire une réponse immunitaire chez un animal auquel il est administré ;
(ii) un composé Pluronic cationique; et
(iii) un ou plusieurs hydrates de carbone polycationiques qui possèdent des propriétés immunostimulantes, dans laquelle l'hydrate de carbone polycationique comprend un dérivé de chitosane alkylé hydrosoluble ou un sel de celui-ci et dans laquelle les particules comprennent des nanosphères de Pluronic cationique qui sont modifiées en surface avec l'hydrate de carbone polycationique

24. Composition pharmaceutique selon la revendication 23, dans laquelle l'hydrate de carbone polycationique est présent sous forme d'un mélange de celui-ci avec un polypeptide cationique, un acide polyaminé cationique ou un composé d'ammonium quaternaire.

25. Composition selon la revendication 24, dans laquelle le chitosane alkylé hydrosoluble est choisi parmi le triméthylchitosane, le chlorure de triméthylchitosane ou le N-carboxyméthylchitosane.

26. Procédé de production d'une composition pharmaceutique selon la revendication 8 ou 9 et selon l'une quelconque des revendications 10 à 21, lorsque selon la revendication 8 ou 9, lequel procédé comprend l'encapsulation de l'agent biologiquement actif dans le premier matériau, en présence de l'hydrate de carbone polycationique.

27. Procédé de production d'une composition pharmaceutique selon la revendication 8 ou 9 et selon l'une quelconque des revendications 10 à 21, lorsque selon la revendication 8 ou 9, lequel procédé comprend la formation d'une émulsion de l'agent biologiquement actif et du premier matériau, en présence de l'hydrate de carbone polycationique, et le versement de l'émulsion résultante dans une seconde phase aqueuse qui contient également l'hydrate de carbone polycationique.

28. Procédé de production d'une composition pharmaceutique selon la revendication 23, lequel procédé comprend la formation d'une nanosphère du composé Pluronic^{®} cationique, le dépôt d'une couche de l'hydrate de carbone polycationique sur celle-ci, et ensuite l'adsorption de l'agent biologiquement actif.

29. Chitosane alkilé hydrosoluble pour utilisation en tant qu'immunostimulant dans le traitement d'infection par un pathogène.
